# FASCICULE DE BREVET EUROPEEN

(11) **EP 0 917 464 B1**
(45) Date de publication et mention de la délivrance du brevet: **12.05.2004**
(21) Numéro de dépôt: 97935643.3
(22) Date de dépôt: 28.07.1997
(51) Int. Cl.: A61K 31/135, A61K 31/55, A61K 31/445, A61K 31/535, A61K 31/40, A61K 31/495, A61K 31/35, C07D 211/14, C07D 211/70, C07C 211/29, C07C 211/41, A61P 35/00

(54) **AMINES POUR LA FABRICATION DE MEDICAMENTS DESTINES A EMPECHER LA PROLIFERATION DE CELLULES TUMORALES**
AMINE ZUR HERSTELLUNG VON MEDIKAMENTEN ZUR HEMMUNG DER TUMORZELLENVERMEHRUNG
USE OF AMINES TO PRODUCE DRUGS FOR PREVENTING TUMOUR CELL PROLIFERATION

(30) Priorité: 29.07.1996 FR 9609531
(43) Date de publication de la demande: 26.05.1999
(73) Titulaire: SANOFI-SYNTHELABO, 75635 Paris Cédex 13 (FR)
(72) Inventeur: BRELIERE, Jean-Claude, F-34090 Montpellier (FR); FERRARA, Pascual, F-31290 Avignolet-Lauragais (FR); LEBOUTEILLER, Christine, F-31320 Pechabou (FR); PAUL, Raymond, F-34980 Saint-Gély du Fesc (FR); ROSENFELD, Jorge, F-31450 Montgiscard (FR); VAN BROECK, Didier, F-34570 Murviel-lès-Montpellier (FR)
(74) Mandataire: Bernasconi, Jean Raymond
(86) Numéro de dépôt international: PCT/FR1997/001409
(87) Numéro de publication internationale: WO 1998/004251

(56) Documents cités:
- EP-A- 0 376 850
- EP-A- 0 461 986
- EP-A- 0 707 004
- FR-A- 2 016 169
- FR-A- 2 132 547
- FR-A- 2 249 659
- FR-A- 2 455 889
- US-A- 4 537 902
- SILVE ET AL.: "The immunosuppressant SR 31747 blocks cell proliferation by inhibiting a steroid isomerase in Saccharomyces cerevisiae" MOL. CELL. BIOL., vol. 16, no. 6, juin 1996, pages 2719-2727, XP000645472
- CASELLAS ET AL.: "Immunopharmacological profile of SR 31747 : In-vitro and in-vivo studies on humoral and cellular responses" J. NEUROIMMUNOL., vol. 52, no. 2, juillet 1994, pages 193-203, XP000645469
- VILNER ET AL.: "Cytotoxic effects of sigma ligands : sigma receptor-mediated alterations in cellular morphology and viability" J. NEUROSCI., vol. 15, no. 1, janvier 1995, pages 117-134, XP000645466
- BRENT ET AL.: "Sigma-binding site ligands inhibit cell proliferation in mammary and colon carcinoma cell lines and melanoma cells in culture" EUR. J. PHARMACOL., vol. 278, no. 2, 15 mai 1995, pages 151-160, XP000645467
- BOURRIE ET AL.: "A sigma ligand, SR 31747A, potently modulates Staphylococcal enterotoxin B-induced cytokine production in mice" IMMMUNOLOGY, vol. 88, no. 3, juillet 1996, pages 389-393, XP002047071

## Description

La presente invention concerne une nouvelle utilisation de la cis N-cyclohexyl N-éthyl-[3-(3-chloro-4-cyclohexylphényl)allyl]amine ainsi que de ses sels pharmaceutiquement acceptables.

La cis N-cyclohexyl N-éthyl-[3-(3-chloro-4-cyclohexylphényl)allyl]amine, également connue sous le code CM 31747 ou SR 31747 et ci-après désignée « SR 31747 », est décrite dans EP 376850 qui fait aussi état de son activité immunosuppressive.

Il a été trouvé que le SR 31747 empêche les cellules cancéreuses de proliférer et que par conséquent, il peut exercer une activité antitumorale.

L'article de P.J BRENT et al. Eur.Pharmacol., 278, 151-160 (1995) fait état de la faculté de certains ligands de site sigma à inhiber, la prolifération cellulaire de lignées cellulaires, carcinomes mammaire et du colon. Il est également précisé dans ce document que les essais au niveau de la prolifération cellulaire varient significativement selon la liaison sigma, la dose utilisée et la ligne cellulaire examinée. Il est ainsi souligné qu'aucune relation n'a pu être établie entre le pouvoir inhibiteur de la croissance cellulaire manifestée par le ligand sigma testé et son affinité pour les sites sigma.

Le document FR-A-2 455 889 propose à titre d'analgésique et d'anti-déprimant des dérivés d'amine. Ce document ne se réfère pas au composé SR 31 747.

Le document FR-A-2 016 169 décrit des dérivés d'acide cyclopropane carboxylique ayant des propriétés anti-inflammatoires et analgésiques.

B. Bourrié et al. rapporte dans l'article publié dans Immunology, 88,389-393 (1996) le SR 31 747 comme possédant une affinité élevée pour les récepteurs sigma exprimés au niveau des lymphocytes.

Toutefois, aucune explication précise n'est donnée pour expliciter ce comportement du SR 31 747 à l'égard de la prolifération lymphocytaire.

Ainsi, la présente invention concerne l'utilisation de la cis N-cyclohexyl N-éthyl-[3-(3-chloro-4-cyclohexylphényl)allyl]amine pour la préparation de compositions pharmaceutiques destinées à s'opposer à la prolifération cellulaire.

Cette détermination peut-être effectuée avec des cellules tumorales choisies de façon appropriée, de préférence parmi des lignées cellulaires qui prolifèrent facilement *in vitro*. par exemple des cellules de myélomes humain, de carcinome de rein ou de poumon humain ou encore sur des cellules de carcinome mammaire.

Dans le contexte de la présente invention, afin de pouvoir effectuer les déterminations dans des conditions standardisées permettant d'obtenir des résultats constants et reproductibles, on a arbitrairement choisi une lignée de cellules de tumeur mammaire humaine « MCF-7 ».

Selon des études biochimiques et pharmacologiques, l'exposition du SR 31747 à des cellules normales, dans les mêmes conditions que celles qui permettent d'obtenir une activité antiproliférative des cellules cancéreuses, ne provoque aucun effet délétère sur tous les critères examinés, tels que par exemple l'intégrité des structures et des fonctions cellulaires ou le maintien de la viabilité.

Ces produits agissent ainsi avec une grande spécificité d'action *vis-à-vis* des cellules tumorales. L'activité antitumorale a été établie sur plusieurs lignées tumorales numaines *in vitro* et *in vivo* chez la souris. Les cellules utilisées sont toutes issues de la collection internationale ATCC. Des cellules MCF-7 ont été utilisées pour réaliser des essais de liaison.

Les membranes ont été préparées comme suit : 10⁹ cellules MCF-7 sont homogénéisées pendant 10 secondes au Polytron® dans 10 ml de tampon Hepes® pH = 7.4 contenant : D-mannitol 210 mM, sucrose 70 mM, EDTA 1 mM, PMSF 0.3 mM. L'homogenat est centrifugé à 650 xg pendant 15 minutes puis le surnageant est prélevé et centrifugé pendant 1 heure à 100 000 xg. Le culot est remis en suspension dans le tampon Tris-HCl pH = 7.4 à la concentration de 1 mg/ml et stocké à - 70° C. La liaison totale est réalisée dans un tube de 5 ml dans lequel on introduit :
50 µl de suspension membranaire contenant 10 à 50 µg de protéines
175 µl de tampon Tris 50 mM pH = 7,4
25 µl de ³H SR 31747 20 nM dans du Tris 50 mM pH = 7,4 + 0,1 % SAB (sérum albumine bovine).

La liaison non spécifique est réalisée en ajoutant 25 µl de SR 31747 10⁻⁵ M dans la solution précédente (volume final 250 µl) puis en incubant pendant 30 minutes à 25° C. Les fractions libres et liées sont séparées en déposant 200 µl sur une colonne de 1 ml de Sephadex® LH 20 puis on compte la radioactivité sur les 2 premiers ml obtenus en éluant la colonne avec du tampon Tris 50 mM pH = 7,4.

Le composé utilisé selon l'invention a montré des activités Cl₅₀ comprises entre 10⁻¹⁰ et 10⁻⁶ M.

Parmi d'autres cellules utilisées, on a cultivé en routine en milieu RPMI additionné de 10 % de sérum de veau foetal (SVF) les lignées suivantes :
myélomes humain U266 et RPMI 8226
carcinome de rein humain 293
carcinome de poumon humain A549
carcinome mammaire humain MCF-7
MCF-7 leucémie lymphocytaire humaine

L acuité antitumorale est mesurée selon la méthode de colorimétrie utilisant le MTT bromure de 3-(4,5-diméthylthiazol-2,5-diphényltétrazolium comme décrit par Mosmann T.. Journ. of Imm. Methods ; 1983, 65, 55.

Ce dosage colorimétrique permet de mesurer quantitativement l'activité antitumorale d'une solution contenant un composé utilisé salon l'invention.

Selon le protocole utilisé, les cellules en suspension (telles que les cellules de myélomes) sont ensemencées à 2.105 cellules/ml en puits de 1 ml en milieu défini. Les cellules adhérentes, (telles que les cellules MCF-7), sont ensemencées à 5.104 cellules/ml en puits de 1 ml en milieu RPMI + 0,5 % SVF pendant une nuit. Le lendemain le tapis cellulaire est lavé deux fois et remplacé par le milieu défini.

Le milieu défini correspond à : RPMI + 10 µg/ml insuline + 10 µg/ml transferrine humaine.

Pour réaliser cet essai, des cellules sont maintenues en présence de la solution contenant un composé utilisé selon l'invention pendant 5 jours, puis la MTT est ajouté dans le milieu de culture.

Le temps 5 jours qui a été sélectionné correspond au temps optimal pour l'activité. À 5 jours de culture on mesura la prolifération les cellules par la test décrit ci-dessus. La densité optique à 570 nm est mesurée. Une coloration bleue se développe dans les puits où les cellules sont encore en vie. L'intensité de la coloration est proportionnelle à la quantité de cellules vivantes.

Les résultats obtenus sont décrits dans le TABLEAU A ci-après.

**TABLEAU A -**

| *Effet du SR 31747 sur différentes lignées tumorales humaines après 5 jours de traitement. Les résultats sont exprimés en pourcentage de prolifération cellulaire par rapport au contrôle non traité pris comme 100 %* | | | | | |
|---|---|---|---|---|---|
| SR 31747 | U266 | RPMI 8226 | MCF-7 | 293 | A549 |
| 10 µM | 11 | 6 | 5 | 7 | 10 |
| 1 µM | 52 | 9 | 34 | 10 | 14 |
| 100 nM | 67 | 54 | 58 | 36 | 38 |
| 10 nM | 82 | 66 | 75 | 43 | 50 |
| 1 nM | 95 | 79 | 83 | 80 | 56 |
| 100 pM | 100 | 91 | 100 | 84 | 88 |
| 10 pM | 100 | 100 | 100 | 100 | 100 |

On voit que des concentrations aussi faibles que 1 nM à 1 µM sont suffisantes pour induire un arrêt de la croissance de 50 % et que cet effet est obtenu sur toutes les cellules tumorales examinées.

L'effet constaté *in vitro* d'inhibition d'une lignée de cellules MCF-7 a été étudié *in vivo* chez la souris « nude », par voie intrapéritonéale à des doses comprises entre 3 et 100 mg/kg dans un modèle selon Néri C., et *al* ; Cancer Research, 1990, *50* : 5892-5897 et selon Berebbi M., *et al* ; Oncogene, 1990, *5* ; 505-509.

Une autre étude a également été réalisée afin de déterminer l'effet du SR 31747 sur la croissance *in vitro* et *in vivo* des cellules épithéliales tumorales mammaires et prostatiques.

L'étude a consisté à évaluer l'activité antitumorale potentielle du SR 31747 sur la croissance d'une variété de lignées épithéliales cancéreuses du sein. L'étude est effectuée *in vitro* sur cultures cellulaires, ainsi qu'*in vivo* après inoculation des lignées mammaires tumorales dans la souris athymique « nude ».

Les lignées cellulaires qui ont été utilisées sont les suivantes :

La lignée MCF-7 hormono-sensible provient d'une effusion pleurale d'un adénocarcinome du sein. Les cellules MCF-7LY2 antioestrogène-résistantes ont été, obtenues à partir de la lignée MCF-7 par pression sélective en présence d'un antioestrogène de haute affinité. Les lignées MCF-7LCC1 et MCF-7LCC2 ont été développèes chez la souris nude, après inoculation des cellules MCF-7. Les deux lignées sont oestrogènes-indépendantes. Les cellules MCF-7LCC2 sont également antioestrogènes-résistantes. La lignée MCF-7AZTD5 est issue de la lignée MCF-7 après transfection stable avec l'oncogène H-ras. Les cellules MCF-7AZTDS sont antioestrogéne-résistantes *in vitro*. L'ensemble de ces lignées est maintenu sur DMEM/F12 (1/1, vol/vol) contenant 10 % de sérum de veau foetal décomplémenté et 16 ng/ml d'insuline humaine. La lignée MCF-7LCC2 est cultivée en présence permanente de 10⁻⁷ M hydroxy-tamoxifène. Les cellules épithéliales mammaires tumorales MDA-MB231 sont oestrogènes et antioestrogènes-insensibles. Elles sont cultivées dans du milieu L15, supplémenté avec 10 % FCS, 1 % d'acides aminés essentiels et 10 µg/ml d'insuline humaine.

Les lignées épithéliales InCaP, PC3 et DU145 sont issues d'adénocarcinomes prostatiques. Seules les cellules LnCaP sont hormono-sensibles. Les trois lignées sont cultivées dans du RPMI supplémenté avec 10 % FCS.

Toutes les lignées sont maintenues à 37° C dans une atmosphère humide air/CO₂ (95 %/5 %). excepté les cellules MDA-MB231 qui sont cultivées en absence de CO₂.

Les lignées cellulaires sont contrôlées régulièrement exemptes de mycoplasmes. L'inoculation et le traitement des animaux ont été effectués comme suit :

Les animaux utilisés sont des souris athymiques « nude » femelles, ovariectomisées ou pas, obtenues à l'âge de 4 semaines (R. Janvier). Avant l'inoculation des cellules mammaires hormonosensibles (MCF-7, MCF-7AZTD5), les animaux reçoivent 10 µl d'une solution éthanotique d'oestradiol 10⁻⁵ M appliquée en percutané. Le traitement est renouvelé trois fois pendant une semaine. Un million de cellules en suspension dans 100 µl de PBS/Ca⁻⁻ sont inoculées en sous-cutané au-dessus de chacune des hanches. Le lendemain, les animaux reçoivent 200 µl de solution d'injection (éthanol/Tween 80/sérum physiologique, 1/1/18, vol/vol) contenant les différents réactifs aux concentrations préalablement définies. Les injections se font par voie intrapéritonéale et sont renouvelées tous les jours.

### RÉSULTATS

### Activité in vitro du SR 31747 sur la prolifération de différentes lignées épithéliales tumorales du sein

Les résultats concernant l'effet du SR 31747 sur la prolifération des cellules épithéliales mammaires en culture sont présentés dans le TABLEAU B. L'activité du SR 31747 a été évaluée en présence de différentes concentrations de sérum. En présence de concentrations réduites de sérum, le SR 31747 exerce une action inhibitrice sur la prolifération de toutes les lignées épithéliales tumorales testées. Des quantités plus élevées de sérum dans les milieux de culture antagonisent partiellement ou totalement activité inhibitrice du SR 31747 sur la croissance cellulaire.

pour une concentration sérique donnée, la capacité du SR 31747 à inhiber la prolifération varie également suivant la lignées considérée. En présence de 0,1 % FCS qui permet à la fois de maintenir une prolifération cellulaire minimale et d'observer une activité conséquente du SR 31747. l'IC₅₀ déterminé pour les lignées MCF-7 hormonosensibles (MCF-7, MCF-7AZ, MCF-7 22HTB) varie de 2.10⁻⁹ M à 7.10⁻³ M. Des valeurs d'IC₅₀ similaires sont obtenues également pour les cellules MDA-MB231 et MCF-7LCC. Par contre, les lignées MCF-7AZTD5 et MCF-7LY2 présentent des sensibilités plus importantes à l'action inhibitrice du SR 31747 puisque les IC₅₀ déterminés sont respectivement de 2.10⁻¹⁰ et 5.10⁻¹² M.

Dans tous les cas, les inhibitions quasi totales de la prolifération cellulaire observées en présence de 10⁻⁶ M et quelquefois 10⁻⁷ M SR 31747 sont, de toute évidence, des effets cytotoxiques.

Les résultats obtenus indiquent également que le SR 31747 est incapable de reverser l'action stimulatrice de l'oestradiol sur la lignée hormonosensible MCF-7.

**TABLEAU B -**

| *Effet du SR 31747 sur la prolifération des cellules épithéliales mammaires*. *Détermination des IC50 après 6 jours de traitement - Milieu changè toutes les 48 heures* | | | | | |
|---|---|---|---|---|---|
| Cellules | Sensibilité | 10 % FBS | 0,5 % FBS | 0,1 % FBS | 0 % FBS |
| MCF7 p23-25 | hormono-sensible | | 2.5 10⁻⁷ M 10⁻⁷ M | 2.10⁻⁹ M 2.10⁻⁸ M | |
| MCF7 RPMI p6-7 | hormono-sensible | | | 7.10⁻⁸ M | 3.10⁻³ M |
| MCF7 22HTB RPMI p+4 | hormono-sensible | | | 2.10⁻⁹ M | |
| LY2 p22-23 | anti-oestrogène résistante | > 10⁻⁷ M 10⁻⁷ M | | 10⁻¹² M 5.10⁻¹² M | |
| MCF7 AZ TD5 | H-ras transfectée anti-oestrogène | 6.10⁻⁷ M | | 2.10⁻¹⁰ M | |
| SN 47 p4 | cellules normales | > 10⁻⁶ M⁷ | 4.10⁻⁷ M | | |
| MCF7 AZ | hormono-sensible | > 10⁻⁶ M | | 2.5 10⁻⁸ M | |
| MCF7 + E2 10-9M | hormono-sensible | | 4.10⁻⁷ M 3.10⁻⁷ M 4.10⁻⁷ M | 5.10⁻¹⁰ M 2.10⁻⁹ M 2.10⁻⁹ M | |
| MDA MB231 p131 | hormono-sansible | 8.10⁻⁷ M 4.10⁻⁷ M | | 2.10⁻⁹ M 2.10⁻⁹ M | |
| LCC1 p23-24 | oestrogène-indépendante | 5 % FBS 10⁻⁶ M | | 2.10⁻⁹ M | |
| LCC2 p26-27 | oestrogène-indépendante anti-oestrogène résistante | 5 % DCC 4.10⁻⁷ M 3.10⁻⁷ M | | 0,1 % DCC 2.10⁻⁹ M 2.10⁻⁹ M | |

### Effet in vivo du SR 31747 sur la fréquence et la taille des tumeurs développées après inoculation de lignées épithéliales mammaires tumorales dans la souris « nude » :

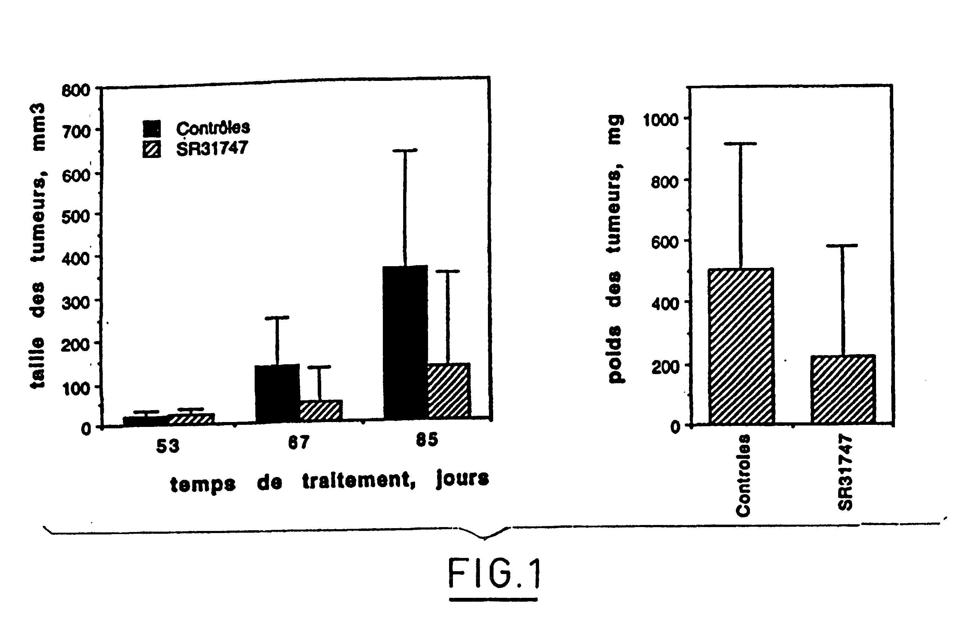

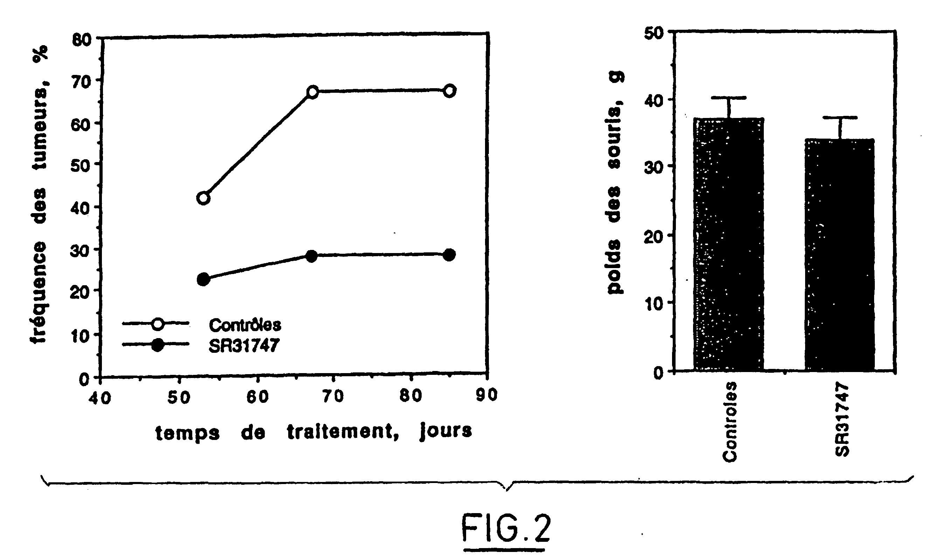

Les FIGURES 1 et 2 indiquent l'effet du SR 31747 sur les tumeurs développées dans la souris « nude » ovariectomisée à partir des cellules MDA-MB231.

Chaque souris reçoit 5.10⁶ cellules MDA-MB231 au-dessus de chaque hanche (soit 2 points d'inoculation par souris). Les animaux sont ensuite traités quotidiennement par voie intrapéritonéale, et reçoivent 500 ug SR 31747 + 0.003 ug E2 (20 souris). Le lot controlé reçoit 0.003 µg E2 (20 souris). La quantité (en pourcentage de tumeurs inoculées) et la taille des tumeurs développées sont déterminées à différents temps au cours du traitement. A la fin du traitement (jour 92), les animaux sont sacrifiés et pesés. Les tumeurs sont prélevées et pesées.

Les FIGURES montrent une tendance du SR 31747 à réduire la taille, le poids et la fréquence des tumeurs développées dans la souris nude ovariectomisée à partir des cellules MDA-MB.231. Cependant, les différences au niveau de la taille et du poids des tumeurs entre les groupes contrôles et SR 31747 n'apparaissent pas significatives, dû à la grande variabilité observée au sein de chaque groupe.

Ainsi, *in vitro*, le SR 31747 présente une activité antiproliférative sur la prolifération de l'ensemble des cellules épithéliales tumorales mammaires étudiées. Cette activité est surtout apparente en présence de concentrations réduites de sérum, et varie en fonction des lignèes considérées. Si, dans la plupart des cas, les IC₅₀ mesurés en 0.1 % FBS varient entre 2.10⁻⁹ M et 2.10⁻⁴ M, on observe cependant une sensibilité extrême des cellules MCF-7LY2 au SR 31747.

Une tendance générale du SR 31747 à réduire à la fois la taille, le poids et la fréquence des tumeurs développées à partir des cellules MDA-MB231 est observée.

L'utilisation du SR 31747 selon l'invention comprend également la préparation de médicaments destinés à la thérapie impliquant le ciblage de produits radiomarqués comme libérateur de radiations à proximité ou dans la tumeur elle-mème, en utilisant le dit composé pour cibler des produits radiomarquès sur la tumeur ou dans son environnement immédiat afin de soumettre les cellules tumorales aux radiations émises par lesdits produits radiomarqués.

L'utilisation du composé selon l'invention peut également comprendre une étape de radiothérapie où les cellules en contact avec le composé subissent l'effet de radiations ionisantes incluant par exemple les rayons gamma, bêta, X ou particules alpha pouvant être délivrées par une source extérieure comme pour les rayons X ou gamma, ou par des radionucléides directement administrés au patient comme décrit par exemple dans Principles of Radiation Therapy in Cancer, Principles and Practice of Oncology, Devita, V.T. *et al*. eds, 4 th ed. J.B. Lippincott Co. Philadelphia, 1993, *15*. 248-275.

Ce composé peut également être administré en association avec d'autres principes actifs anticancéreux bien connus de l'homme de l'art.

Le composé SR 31747 peut être utilisé en thérapie dans tous les processus pathologiques qui entrainent la prolifération des cellules tumorales. Cette prolifération cellulaire peut être soit hormono-sensible ou hormono-insensible.

Plus précisément des applications cliniques pour lesquelles ont peut envisager l'utilisation de ce composé comprennent les maladies découlant d'une prolifération cellulaire, en particulier des glioblastomes, des neuroblastomes, des lymphômes. des myélomes, de la leucémie, des carcinomes du colon, colorectal, épithélial, hépatique. pulmonaire, mammaire, ovarien, pancréatique ou encore de la prostate.

À ces fins, le composé SR 31747 ainsi que ses sels pharmaceutiquement acceptables peut être utilisé pour la préparation de compositions pharmaceutiques par voie orale, parentérale, sublinguale, transdermique ou topique. Ces compositions pharmaceutiques renferment au moins un des produits ci-dessus, en association avec un véhicule pharmaceutiquement inerte.

Plus particulièrement, la présente invention concerne, selon un autre de ses aspects, des compositions pharmaceutiques contenant, en tant que principe actif, le SR 31747 ou un de ses sels pharmaceutiquement acceptables.

Les acides tant organiques qu inorganiques peuvent être utilisés pour former des sels d'addition d'acides du SR 31747 qui soient non toxiques et pharmaceutiquement acceptables, notamment les acides sulfurique, nitrique, phosphorique, chlorhydrique, citrique, acétique, lactique, tartrique, parnoïque, éthanédisulfonique, méthanesulfonique, succinique, cyclohyxylsulfonique, fumarique. maléique et benzoïque.

En ce qui concerne l'administration par voie orale ou sublinguale, on utilise en particulier des comprimés, simples ou dragéifiés, des gélules, des granules eventuellement à libération retardée, des gouttes ou encore des liposomes. En ce qui concerne l'administration par voie intraveineuse, sous-cutanée ou intramusculaire, on a recours à des solutions stériles ou stérilisables en particulier pour perfusion veineuse, tandis que l'on peut réaliser des patches conventionnels pour l'administration par voie transdermique. Pour l'utilisation topique on peut utiliser des crèmes ou des lotions à étendre sur la peau.

Les compositions pharmaceutiques selon la présente invention peuvent être préparées selon des méthodes usuelles bien connues dans le domaine de la technique pharmaceutique.

Le principe actif peut être incorporé dans les excipients habituellement employés dans ces compositions pharmaceutiques, tels que le talc, la gomme arabique, le lactose, l'amidon, la stéarate de magnésium, les véhicules aqueux ou non, les corps gras d'origine animale ou végétale, les dérivés paraffiniques, les glycols, les différents agents mouillants, dispersants, ou émulsifiants, les conservateurs, etc.

Les compositions pharmaceutiques de invention peuvent avantageusement contenir le SR 31747 ou l'un de ses sels d'addition pharmaceutiquement acceptables en association avec un ou plusieurs autres médicaments connus et communément utilisés pour les mêmes indications thérapeutiques.

La quantité de principe actif à administrer par jour, selon la méthode de la présente invention, dépend de la particularité de l'indication thérapeutique, de la gravité des affections à traiter, ainsi que du poids du malade et de la voie d'administration.

Pour une administration systématique, la dose globale chez l'homme varie généralement entre 1 et 100 mg par jour, par exemple de 2 à 50 mg, et plus convenablement de 3 à 40 mg par jour.

Des formes unitaires de dosage pour l'administration systémique comprendront généralement de 3 à 50 mg (à savoir 3, 5, 10, 20, 30, 40, et 50 mg de produit). Ces doses unitaires seront administrées normalement une ou plusieurs fois par jour, de préférence une à trois fois par jour.

Pour l'administration topique, les compositions pharmaceutiques contiennent généralement de 0,0001 à 10 % de principe actif et de préférence de 0,01 à 5 %.

## Revendications

1. Utilisation de la cis N-cyclohexyl N-éthyl-[3-(3-chloro-4-cyclohexylphényl)allyl]amine ou un de ses sels ou solvates pharmaceutiquement acceptables pour la préparation de compositions pharmaceutiques destinées au traitement de maladies découlant d'une prolifération cellulaire.

2. Utilisation selon la revendication 1, **caractérisée en ce que** la prolifération cellulaire est hormono-sensible.

3. Utilisation selon la revendication 1, **caractérisée en ce que** la prolifération cellulaire est hormono-insensible.

4. Utilisation selon l'une des revendications 1 à 3 pour la préparation de compositions pharmaceutiques destinées au traitement des glioblastomes, des neuroblastomes, des lymphomes, des myélomes, de la leucémie, des carcinomes du colon, colorectaux, épithéliaux, hépatiques, pulmonaires, mammaires, ovariens, pancréatiques ou de la prostate.

5. Composition pharmaceutique destinée au traitement de maladies découlant d'une prolifération cellulaire comprenant la cis N-cyclohexyl N-éthyl-[3-(3-chloro-4-cyclohexylphényl)allyl]amine ou un de ses sels ou solvates pharmaceutiquement acceptables.

6. Composition pharmaceutique selon la revendication 5, **caractérisée en ce qu'**elle est destinée au traitement des glioblastomes, des neuroblastomes, des lymphomes, des myélomes, de la leucémie, des carcinomes du colon, colorectaux, épithéliaux, hépatiques, pulmonaires, mammaires, ovariens, pancréatiques ou de la prostate.

## Patentansprüche

1. Verwendung von cis-N-Cyclohexyl-N-ethyl-[3-(3-chlor-4-cyclohexylphenyl)-allyl]-amin oder eines seiner pharmazeutisch annehmbaren Salze oder Solvate zur Herstellung von pharmazeutischen Zubereitungen für die Behandlung von Krankheiten. die mit einer Zellproliferation einhergehen.

2. Verwendung nach Anspruch 1, **dadurch gekennzeichnet, daß** die Zellproliferation Hormon-empfindlich ist.

3. Verwendung nach Anspruch 1, **dadurch gekennzeichnet, daß** die Zellproliferation Hormon-unempfindlich ist.

4. Verwendung nach einem der Ansprüche 1 bis 3 für die Herstellung von pharmazeutischen Zubereitungen für die Behandlung von Glioblastomen, Neuroblastomen, Lymphomen, Myleomen, Leukämie, Darm-, Kolorektal-, Epithelial-, Leber-, Lungen-, Brust-, Ovarial-, Pankreas- oder Prostatakrebs.

5. Pharmazeutische Zubereitung für die Behandlung von Erkrankungen, die mit einer Zellproliferation einhergehen, umfassend cis-N-Cyclohexyl-N-ethyl-[3-(3-chlor-4-cyclohexylphenyl)-allyl]-amin oder eines seiner pharmazeutisch annehmbaren Salze oder Solvate.

6. Pharmazeutische Zubereitung nach Anspruch 5, **dadurch gekennzeichnet, daß** sie zur Behandlung von Glioblastomen, Neuroblastomen, Lymphomen, Myelomen, der Leukämie, Darm-, Kolorektal-, Epithelial-, Leber-, Lungen-, Brust-, Ovarial-, Pankreas- oder Prostatakrebs bestimmt ist.

## Claims

1. Use of cis N-cyclohexyl-N-ethyl-[3-(3-chloro-4-cyclohexylphenyl)allyl]amine or a pharmaceutically acceptable salt or solvate thereof for the preparation of pharmaceutical compositions for treatment of diseases resulting from cell proliferation.

2. Use according to claim 1, **characterised in that** the cell proliferation is hormone-sensitive.

3. Use according to claim 1, **characterised in that** the cell proliferation is hormone-insensitive.

4. Use according to one of claims 1 to 3 for the preparation of pharmaceutical compositions for the treatment of glioblastomas, neuroblastomas, lymphomas, myelomas, leukaemia, carcinomas of the colon, colorectal, epithelial, hepatic, pulmonary, mammary, ovarian, pancreatic or prostate carcinomas.

5. pharmaceutical composition for the treatment of diseases resulting from cell proliferation, comprising *cis* N-cyclohexyl-N-ethyl-[3-(3-chloro-4-cyclohexylphenyl)allyl]amine or a pharmaceutically acceptable salt or solvable thereof.

6. Pharmaceutical composition according to claim 5, **characterised in that** it is for the treatment of glioblastomas, neuroblastomas, lymphomas, myelomas, leukaemia, carcinomas of the colon, colorectal, epithelial, hepatic, pulmonary, mammary, ovarian, pancreatic or prostate carcinomas.
